# EUROPÄISCHE PATENTANMELDUNG

(11) **EP 4 226 965 A1**
(43) Veröffentlichungstag der Anmeldung: **16.08.2023**
(21) Anmeldenummer: 22020051.3
(22) Anmeldetag: 15.02.2022
(51) Int. Cl.: A61M 60/178, A61M 60/232, A61M 60/221, A61M 60/422, A61M 60/804, A61M 60/814

(54) **BLUTPUMPE ZUR UNTERSTÜTZUNG DER HERZLEISTUNG**

(71) Anmelder: Medizinische Universität Wien, 1090 Wien (AT); Charité - Universitätsmedizin Berlin, 10117 Berlin (DE)
(72) Erfinder: Granegger, Marcus, A-2380 Perchtoldsdorf (AT); Narayanaswamy, Krishnaraj, A-1190 Wien (AT); Escher, Andreas, A-1160 Wien (AT); Zimpfer, Daniel, A-1190 Wien (AT); Kertzscher, Ulrich, D-10713 Berlin (DE)
(74) Vertreter: Keschmann, Marc

(57) **Zusammenfassung**

Bei einer Blutpumpe zur Unterstützung der Herzleistung, umfassend ein Gehäuse mit einem Gehäuseeinlass (1) und einem Gehäuseauslass (2), wobei das Gehäuse ein erstes Pumpengehäuse (4) und ein zweites Pumpengehäuse (9) ausbildet, einen Rotor (14) der in dem Gehäuse gelagert ist und um eine Achse (3) drehbar ist, wobei der Rotor (14) eine erste Pumpstufe mit einem in dem ersten Pumpengehäuse (4) angeordneten ersten Laufrad (5) und eine zweite Pumpstufe mit einem in dem zweiten Pumpengehäuse (9) angeordneten zweiten Laufrad (10) umfasst, wobei die erste und die zweite Pumpstufe jeweils als Radial-bzw. Diagonalpumpe ausgebildet sind und nacheinander von dem vom Gehäuseeinlass (1) zum Gehäuseauslass (2) zu pumpenden Blut durchströmbar sind, sind das erste Pumpengehäuse (4) und das zweite Pumpengehäuse (9) über einen Kanal (7) verbunden sind, der radial auberhalb eines zwischen dem ersten und dem zweiten Pumpengehäuse (4,9) angeordneten Mittelabschnitts (15) des Gehäuses verläuft.

## Beschreibung

Die Erfindung betrifft eine Blutpumpe zur Unterstützung der Herzleistung, umfassend ein Gehäuse mit einem Gehäuseeinlass und einem Gehäuseauslass, wobei das Gehäuse ein erstes Pumpengehäuse und ein zweites Pumpengehäuse ausbildet, einen Rotor der in dem Gehäuse gelagert ist und um eine Achse drehbar ist, wobei der Rotor eine erste Pumpstufe mit einem in dem ersten Pumpengehäuse angeordneten ersten Laufrad und eine zweite Pumpstufe mit einem in dem zweiten Pumpengehäuse angeordneten zweiten Laufrad umfasst, wobei die erste und die zweite Pumpstufe jeweils als Radial bzw. Diagonalpumpe ausgebildet sind und nacheinander von dem vom Gehäuseeinlass zum Gehäuseauslass zu pumpenden Blut durchströmbar sind.

Herzinsuffizienz (HF) ist die häufigste Todesursache in den westlichen Ländern. Moderne Geräte zur mechanischen Kreislaufunterstützung (MCS) fördern das Überleben und verbessern die Lebensqualität vieler HF-Patienten. Rotodynamische Blutpumpen (RBPs) stellen die überwiegende Mehrheit der im letzten Jahrzehnt implantierten MCS-Geräte dar, wobei die Überlebensrate nach zwei Jahren über 70 % beträgt. Dennoch bleiben die klinischen Ergebnisse durch schwere Nebenwirkungen eingeschränkt, von denen die meisten durch suboptimale Interaktionen zwischen Pumpensystem und Blut hervorgerufen werden. Nur 20 % aller RBP-Empfänger bleiben ein Jahr nach der Implantation frei von lebensbedrohlichen schweren Nebenwirkungen, einschließlich Blutungen oder Schlaganfällen. Folglich ist die Lebensqualität dieser Patienten weiterhin stark eingeschränkt und die Kosteneffizienz dieser Therapie ist noch nicht erreicht.

Moderne implantierbare Blutpumpen sind für erwachsene Patienten konzipiert, die an einer Herzinsuffizienz vom HFrEF-Typ leiden, d.h. einer Herzinsuffizienz mit reduzierter linksventrikulärer Ejektionsfraktion. Solche Patienten benötigen implantierbare Blutpumpen, die einen vergleichsweise hohen mittleren Fluss (4-5 l/min) bereitstellen. Der Einsatz solcher Pumpen in Anwendungen mit niedrigem Durchfluss (1-2,5 l/min) birgt das Risiko eines erhöhten Bluttraumas und einer erhöhten Komplikationsrate.

Es besteht ein Bedarf an einer hämokompatiblen implantierbaren Blutpumpe, die für Patienten geeignet ist, bei denen die Pumpe einen vergleichsweise niedrigen Blutfluss bereitstellen soll. Solche Fälle umfassen:
(i) pädiatrische HF-Patienten,
(ii) HFrEF-Patienten mit einem geringeren Schweregrad der Herzinsuffizienz, insbesondere Herzinsuffizienz im NYHA-Stadium 3-3b, um das Fortschreiten der Symptome zu stoppen oder umzukehren,
(iii) die große Population von HF-Patienten mit erhaltener Ejektionsfraktion (HFpEF), einer Population, für die derzeit keine andere wirksame Langzeitbehandlungsoption verfügbar ist.

Der Artikel von Thamsen et al: "A two-stage rotary blood pump design with potentially lower blood trauma: a computational study" in Int J Artif Organs, 2016 Jun 15; 39(4):178-83, beschreibt eine zweistufige Blutpumpe zur Reduzierung von Bluttraumata bei Anwendungen, bei denen die Pumpe einen niedrigen Blutfluss bereitstellen soll. Hierbei wird der Druckaufbau auf zwei hintereinander durchströmbare Pumpenstufen aufgeteilt, was zu einer Reduktion der Umfangsgeschwindigkeiten des Impellers und somit reduzierter Blutschädigung führt.. Insbesondere bei kleinen Flüssen und vergleichsweise hohen Druckdifferenzen (niedrige spezifische Drehzahl) kann eine zweistufige Bauform zu einem effizienteren und blutschonenderen Betrieb führen. Die in Thamsen et al vorgeschlagene Ausführungsform ermöglicht jedoch keine Miniaturisierung in einem für die Implantation erforderlichen Ausmaß.

Zusammenfassend ist festzuhalten, dass derzeit verfügbare Blutpumpen entweder zu groß für eine Implantation sind und/oder bei niedrigeren Blutflüssen ein erhöhtes Risiko für Bluttraumata und Komplikationsraten aufweisen, um Kinder bzw. Erwachsene mit weniger fortgeschrittenem Herzfehler zu versorgen. Diese Beobachtungen können dadurch erklärt werden, dass Betriebsbedingungen mit geringen Pumpendurchflüssen bei vergleichsweise hohen Drücken zu Pumpenkonstruktionen mit niedrigen spezifischen Drehzahlen führen - eine Schlüsselgröße, die anzeigt, dass diese Betriebsbedingungen mit hohen hydraulischen Wirkungsgraden nicht vereinbar sind. Folglich sind vergleichsweise hohe Leistungen erforderlich, um Druck und Strömung zu erzeugen. Diese verringerte Effizienz spiegelt sich in erhöhten Umfangsgeschwindigkeiten wider, welche wiederum zu erhöhten Bluttraumata führen können.

Die vorliegende Erfindung zielt daher darauf ab, eine miniaturisierte implantierbare Blutpumpe zu schaffen, die das Potenzial besitzt, hämokompatibilitätsbedingte Komplikationen zu reduzieren. Insbesondere soll die Erfindung dem Bedarf einer Niedrigfluss-Blutpumpe für i) die pädiatrische Population mit Herzinsuffizienz und ii) erwachsene Patienten mit Herzinsuffizienz mit entweder reduzierter (HFrEF) oder erhaltener (HFpEF) Ejektionsfraktion Rechnung tragen.

Zur Lösung dieser Aufgabe sieht die Erfindung bei einer Blutpumpe der eingangs genannten Art im Wesentlichen vor, dass das erste Pumpengehäuse und das zweite Pumpengehäuse über einen Kanal verbunden sind, der radial außerhalb eines zwischen dem ersten und dem zweiten Pumpengehäuse angeordneten Mittelabschnitts des Gehäuses verläuft. Dadurch, dass das von der ersten Pumpstufe zur zweiten Pumpstufe zu transportierende Blut nicht entlang des Rotors, d.h. innerhalb des Mittelabschnitts des Gehäuses geleitet wird, sondern außerhalb des Mittelabschnitts, wird eine in axialer Richtung kurze Bauweise der Blutpumpe ermöglicht. Insbesondere kann der Mittelabschnitt aufgrund der über den externen Kanal erfolgenden Blutführung als Rotorlager genutzt werden. Diesbezüglich sieht eine bevorzugte Ausbildung der Erfindung vor, dass der Rotor in dem Mittelabschnitt des Gehäuses drehbar gelagert ist. Besonders bevorzugt bildet der Mittelabschnitt des Gehäuses mit dem Rotor ein hydrodynamisches Lager aus. Der Mittelabschnitt ist hierbei mit einer bevorzugt zylindrischen Innenoberfläche ausgebildet, welche mit einer zylindrischen Rotorfläche zur Ausbildung des hydrodynamisches Lagers zusammenwirkt. Der Lagerspalt beträgt beispielsweise 50-150pm.

Dadurch, dass die Blutpumpe zwei seriell durchströmbare Pumpstufen aufweist, wird der Druckaufbau über die zwei Stufen verteilt und erlaubt im Vergleich zu einstufigen Pumpen den Betrieb bei bis zu 30% niedrigeren Drehzahlen.

In strömungstechnisch günstiger Weise kann vorgesehen sein, dass der Kanal den Mittelabschnitt des Gehäuses schraubenlinienförmig umgibt, wobei der Drehsinn der Schraubenlinienform an die Drehrichtung des Rotors bzw. der Laufräder angepasst ist. Der schraubenlinienförmige Kanal kann sich entlang eines Winkels von beispielsweise 150-210° um die Pumpenachse erstrecken.

Eine strömungstechnisch günstige und platzsparende Anordnung des Kanals kann gemäß einer bevorzugten Ausbildung der Erfindung erreicht werden, wenn das erste Pumpengehäuse einen das erste Laufrad umgebenden ringförmigen Bereich (Auslassgehäuse/Volute) aufweist, von welchem der Kanal tangential wegführt.

Eine strömungsgünstige Ableitung des von der Pumpe geförderten Bluts gelingt bevorzugt dadurch, dass das zweite Pumpengehäuse einen das zweite Laufrad umgebenden ringförmigen Bereich aufweist (Auslassgehäuse/Volute), von welchem der Gehäuseauslass tangential wegführt.

Weiters kann das zweite Pumpengehäuse einen in axialer Richtung vom zweiten Laufrad beabstandeten Einlassgehäuseabschnitt aufweisen, in welchen der Kanal tangential einmündet.

Hinsichtlich des elektrischen Antriebs der Blutpumpe sieht eine bevorzuge Ausbildung vor, dass der Rotor mit einem den Mittelabschnitt des Gehäuses umgebenden, Motorwicklungen aufweisenden Stator zur Ausbildung eines elektrischen Motors zusammenwirkt. Der Rotor kann hierbei mit Permanentmagneten versehen sein. Die Pumpe wird somit von einem elektromagnetischen Motor angetrieben, der um den hydrodynamischen Lagerbereich herum integriert ist. Gleichzeitig wird durch das Zusammenwirken des Stators mit dem Rotor eine axiale Aufhängung des Rotors auf Basis magnetischer Reluktanzkräfte ermöglicht, was eine miniaturisierte Realisierung der Blutpumpe begünstigt.

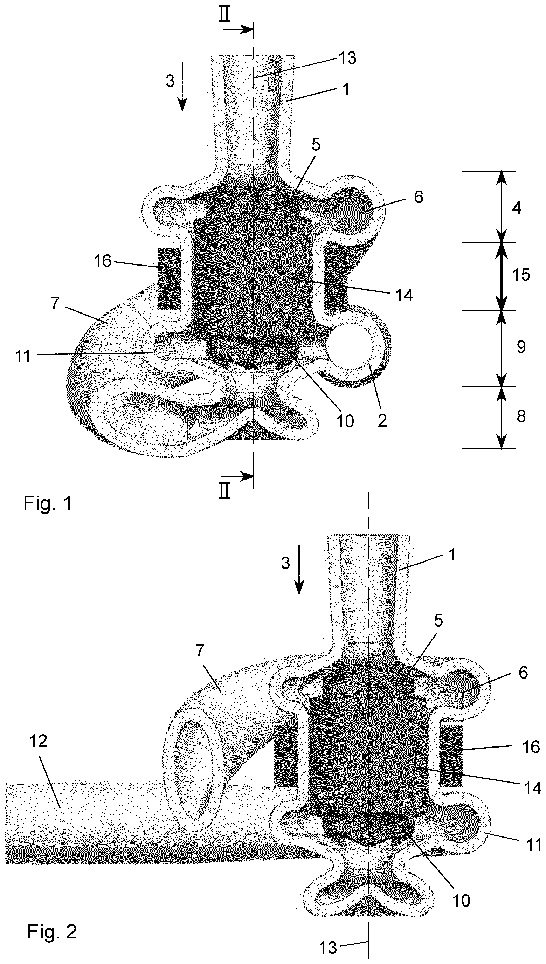

Die Erfindung wird nachfolgend anhand eines in der Zeichnung schematisch dargestellten Ausführungsbeispiels näher erläutert. In dieser zeigen Fig. 1 einen Axialschnitt einer erfindungsgemäßen Blutpumpe und Fig. 2 einen Axialschnitt entlang der Linie II-II der Fig. 1.

Die Blutpumpe umfasst ein Gehäuse, das ein in Flussrichtung 3 leicht konvergierendes Einlassrohr 1 und einen Auslass 2 aufweist. Das durch das Einlassrohr 1 angesaugte Blut durchläuft zuerst eine erste Pumpstufe und danach eine zweite Pumpstufe. Die erste Pumpstufe umfasst eine erstes Pumpengehäuse 4, in dem ein erstes Laufrad 5 rotierend angeordnet ist. Das Laufrad 5 treibt das Blut radial/diagonal nach außen in einen das erste Laufrad 5 umgebenden ringförmigen Bereich 6, von welchem ein Kanal 7 tangential wegführt. Über den Kanal 7 gelangt das Blut in einen Einlassgehäuseabschnitt 8, von welchem es zur zweiten Pumpstufe fließt, in welcher ein in einem zweiten Pumpengehäuse 9 angeordnetes zweites Laufrad 10 rotiert. Das zweite Laufrad 10 treibt das Blut radial/diagonal nach außen in einen das zweite Laufrad 10 umgebenden ringförmigen Bereich 11 des zweiten Pumpengehäuses 9, von welchem der Gehäuseauslass 2 bzw. das Auslassrohr 12 tangential wegführt.

Die erste und die zweite Pumpstufe sind somit entlang der Pumpenachse 13 hintereinander angeordnet, wobei das erste Laufrad 5 und das zweite Laufrad 10 am selben Rotor 14 angeordnet sind, der in einem zwischen dem ersten Pumpengehäuse 4 und dem zweiten Pumpengehäuse 9 angeordneten Mittelabschnitt 15 des Gehäuses unter Ausbildung eines hydrodynamischen Lagers rotierend gelagert ist. Um den Mittelabschnitt 15 des Gehäuses sind Motorwicklungen 16 angeordnet, die mit dem Rotor 14 zur Ausbildung eines elektrischen Motors zusammenwirken. Gleichzeitig wird dadurch eine axiale Positionierung des 14 Rotors auf Basis magnetischer Reluktanzkräfte ermöglicht.

Weiters ist ersichtlich, dass der das erste Pumpengehäuse 4 und das zweite Pumpengehäuse 9 verbindende Kanal 7 radial außerhalb des Mittelabschnitts 15 des Gehäuses verläuft.

Die axial aufeinanderfolgende Anordnung der beiden Pumpstufen in Kombination mit einem in radialer Richtung wirkenden hydrodynamischen Lager und einer Reluktanzmagnetaufhängung in axialer Richtung ermöglicht eine miniaturisierte Realisierung der Blutpumpe. Die Abmessungen des Pumpenkörpers (ohne Einlass- und Auslassrohr) können ca. 2,5x2,5x2,5cm oder weniger betragen.

Alternativ könnte statt des hydrodynamischen Lagers ein Magnetlager oder ein mechanisches Lager verwendet werden.

## Patentansprüche

1. Blutpumpe zur Unterstützung der Herzleistung, umfassend ein Gehäuse mit einem Gehäuseeinlass (1) und einem Gehäuseauslass (2), wobei das Gehäuse ein erstes Pumpengehäuse (4) und ein zweites Pumpengehäuse (9) ausbildet, einen Rotor (14) der in dem Gehäuse gelagert ist und um eine Achse (13) drehbar ist, wobei der Rotor (14) eine erste Pumpstufe mit einem in dem ersten Pumpengehäuse (4) angeordneten ersten Laufrad (5) und eine zweite Pumpstufe mit einem in dem zweiten Pumpengehäuse (9) angeordneten zweiten Laufrad (10) umfasst, wobei die erste und die zweite Pumpstufe jeweils als Radial- oder Diagonalpumpe ausgebildet sind und nacheinander von dem vom Gehäuseeinlass (1) zum Gehäuseauslass (2) zu pumpenden Blut durchströmbar sind, **dadurch gekennzeichnet, dass** das erste Pumpengehäuse (4) und das zweite Pumpengehäuse (9) über einen Kanal (7) verbunden sind, der radial außerhalb eines zwischen dem ersten und dem zweiten Pumpengehäuse (4,9) angeordneten Mittelabschnitts (15) des Gehäuses verläuft.

2. Blutpumpe nach Anspruch 1, **dadurch gekennzeichnet, dass** der Kanal (7) den Mittelabschnitt (15) des Gehäuses schraubenlinienförmig umgibt.

3. Blutpumpe nach Anspruch 1 oder 2, **dadurch gekennzeichnet, dass** das erste Pumpengehäuse (4) einen das erste Laufrad (5) umgebenden ringförmigen Bereich (6) aufweist, von welchem der Kanal (7) tangential wegführt.

4. Blutpumpe nach Anspruch 1, 2 oder 3, **dadurch gekennzeichnet, dass** das zweite Pumpengehäuse (9) einen das zweite Laufrad (10) umgebenden ringförmigen Bereich (11) aufweist, von welchem der Gehäuseauslass (2) tangential wegführt.

5. Blutpumpe nach einem der Ansprüche 1 bis 4, **dadurch gekennzeichnet, dass** das zweite Pumpengehäuse (9) einen in axialer Richtung vom zweiten Laufrad (10) beabstandeten Einlassgehäuseabschnitt (8) aufweist, in welchen der Kanal (7) tangential einmündet.

6. Blutpumpe nach einem der Ansprüche 1 bis 5, **dadurch gekennzeichnet, dass** der Rotor (14) in dem Mittelabschnitt (15) des Gehäuses drehbar gelagert ist.

7. Blutpumpe nach Anspruch 6, **dadurch gekennzeichnet, dass** der Mittelabschnitt (15) des Gehäuses mit dem Rotor (14) ein hydrodynamisches Lager ausbildet.

8. Blutpumpe nach Anspruch 6 oder 7, **dadurch gekennzeichnet, dass** der Rotor (14) mit einem den Mittelabschnitt (15) des Gehäuses umgebenden, Motorwicklungen (16) aufweisenden Stator zur Ausbildung eines elektrischen Motors zusammenwirkt.
